(19) Europäisches Patentamt / European Patent Office / Office européen des brevets

(11) **EP 4 475 921 B1**

(12) **EUROPÄISCHE PATENTSCHRIFT**

(45) Veröffentlichungstag und Bekanntmachung des Hinweises auf die Patenterteilung:
**06.05.2026   Patentblatt 2026/19**

(21) Anmeldenummer: **23705212.1**

(22) Anmeldetag: **08.02.2023**

(51) Internationale Patentklassifikation (IPC):
***A61M 13/00*** *(2006.01)*

(52) Gemeinsame Patentklassifikation (CPC):
**A61M 13/003; A61M 13/00;** A61M 5/16859;
A61M 2202/0468; A61M 2205/18; A61M 2205/3334;
A61M 2205/3344; A61M 2205/50; A61M 2210/1017

(86) Internationale Anmeldenummer:
**PCT/EP2023/053125**

(87) Internationale Veröffentlichungsnummer:
**WO 2023/152186 (17.08.2023 Gazette 2023/33)**

(54) **INSUFFLATIONSVORRICHTUNG MIT NEUARTIGER DRUCKMESSUNG**

INSUFFLATION APPARATUS WITH NOVEL PRESSURE MEASUREMENT

APPAREIL D'INSUFFLATION AVEC NOUVELLE MESURE DE PRESSION

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **08.02.2022   DE 102022000487**

(43) Veröffentlichungstag der Anmeldung:
**18.12.2024   Patentblatt 2024/51**

(73) Patentinhaber: **W.O.M. World of Medicine GmbH 10587 Berlin (DE)**

(72) Erfinder:
- **STIER, Henrik**
  **13086 Berlin (DE)**
- **CARSTENS, Jan Hendrik**
  **10409 Berlin (DE)**

(74) Vertreter: **Gulde & Partner Patent- und Rechtsanwaltskanzlei mbB Berliner Freiheit 2 10785 Berlin (DE)**

(56) Entgegenhaltungen:
DE-U1- 202004 021 703      US-A- 5 908 402
US-A1- 2021 290 864

EP 4 475 921 B1

## Beschreibung

Die vorliegende Erfindung betrifft einen Insufflator mit einer neuartigen Druckmessvorrichtung. Durch die neue Vorrichtung ist es möglich, den Druck in der Körperhöhle sicher zu messen und gleichzeitig sicherzustellen, dass die Gaszuführleitungen frei sind.

## Hintergrund und Stand der Technik

Insufflatoren sind in mannigfaltiger Ausführung aus dem Stand der Technik bekannt. Insufflatoren weisen üblicherweise einen Schlauch auf, durch den ein medizinisches Gas in eine Körperhöhle (z. B. ein Abdomen) eingeleitet wird. Das Gas erzeugt einen Überdruck, welches die Körperhöhle aufdehnt, damit ausreichend Platz für die visuelle Inspektion bzw. den therapeutischen Eingriff besteht. Optionale Ausführungsformen ermöglichen das Absaugen des Gases aus dem Bauchraum über einen zweiten Schlauch. Derartige Ausführungsformen werden insbesondere bei therapeutischen Eingriffen mittels Elektrochirurgie oder Laser genutzt, um gesundheitsschädliche Rauchgase abzuführen und zu filtern.

In der Praxis hat es sich als schwierig herausgestellt, den Druck in der Körperhöhle präzise zu bestimmen. Auch für die Druckbestimmung in der Körperhöhle sind verschiedene Ausgestaltungen von Messvorrichtungen bekannt. Die Möglichkeit, den Druck durch einen Drucksensor in der Körperhöhle selbst zu messen, hat den Nachteil, dass neben den notwendigen medizinischen Instrumenten auch der Drucksensor in der Körperhöhle positioniert sein muss. Dies bedeutet einerseits ein zusätzliches Element in der Körperhöhle, mit dazugehörigen Anschlüssen, welches Platz wegnimmt und gleichzeitig eine Infektionsgefahr darstellt, andererseits werden hierdurch höhere Kosten für den Drucksensor erzeugt, weil dieser entweder aufwändig gereinigt und sterilisiert werden oder als Einwegartikel ausgelegt sein muss.

Etabliert haben sich daher Ausführungsformen, bei denen der Drucksensor sich nicht in der Körperhöhle selbst befindet, sondern in einem der Schläuche, mehr oder weniger beabstandet zur Körperhöhle. Der prinzipbedingte Nachteil dieser Sensoranordnung ist, dass es einen Druckunterschied zwischen der Körperhöhle und der Messposition (z. B. im Schlauch) gibt, bedingt durch den Schlauch, den Trokar und möglicherweise weitere Vorrichtungen. Die Bestimmung dieses Druckverlustes ist nicht einfach und Gegenstand vieler Untersuchungen. Je weiter weg der Drucksensor von der Körperhöhle liegt, desto größer sind die Abweichungen des gemessenen Drucks vom Druck in der Körperhöhle. Je näher der Drucksensor an die Gaszuführeinrichtung (z. B. ventilgesteuerte Pumpe) oder einer Absaugpumpe kommt, desto größer sind die Abweichungen bedingt durch die Aktivität der Pumpen (z.B. Druckpulse) bzw. der Ventile. Die Effekte werden darüber hinaus überlagert von Fehlern, die dadurch entstehen, dass die Schläuche verschlossen sind und keinen Fluidfluss zur oder von der Körperhöhle erlauben. Eine solche Blockade kann durch einen abgeknickten Schlauch gegeben sein oder dadurch, dass die patientenseitige Öffnung durch Körpergewebe verschlossen ist. In einem solchen Fall des Schlauchverschlusses misst der Drucksensor lediglich den Druck im Schlauch, nicht den Druck in der Körperhöhle, was zu erheblichen Sicherheitsbedenken führt.

Aus der DE 202004 021703 U1 bekannt ist ferner eine Vorrichtung zur Abgabe von Substanzen (z.B. Chemotherapeutika) in eine insufflierte Körperhöhle, die allerdings keine Vermeidung der eingangs angesprochenen Messfehler, insbesondere keine Feststellung einer Schlauchokklusion, erlaubt.

US 5,908,402A beschreibt ein Verfahren und eine Vorrichtung zur Erkennung von Rohrverstopfungen in einem elektrochirurgischen Argon-System.

Um die oben genannten Nachteile bisheriger Druckmesssysteme zu überwinden, hat sich die Aufgabe gestellt, einen Insufflator zur Verfügung zu stellen, der eine präzise Druckmessung gewährleistet und eine gegebenenfalls auftretende Schlauchokklusion zuverlässig erkennt.

Die vorliegende Erfindung betrifft daher einen Insufflator für die minimalinvasive Chirurgie, enthaltend

    a) Gasanschluss für eine Gasflasche oder Hausgas (1)
    b) erste Druck- und Durchflussregeleinheit (2) ausgestattet mit Proportionalventil,
    c) Zuführleitung (3) mit einem Querschnitt von 5,5 bis 15 mm, ausgelegt für einen Gasfluss von 0-50 L/min bei 2-3 bar, mit erstem steuerbarem Ventil (4), erstem Gasdrucksensor (5), erstem Gasflusssensor (6) und Anschluss an einen ersten Trokar (7) in eine Kavität (8),
    d) Sensorleitung (9) mit einem Querschnitt von 2 bis 5 mm mit einer optionalen Drossel (10), ausgelegt für einen Gasfluss von weniger als 1 L/min bei 2-3 bar, mit einem zweiten steuerbaren Ventil (11) und einem zweiten Gasdrucksensor (12) und Anschluss an einen zweiten Trokar (13) in die Kavität (8),
    e) Recheneinheit (14) zur Steuerung der Insufflation und Auswertung der Messdaten von erstem Gasdrucksensor (5) und zweitem Gasdrucksensor (12),

wobei die Sensorleitung (9) an die erste Druck- und Durchflussregeleinheit (2) angeschlossen ist und wobei das zweite steuerbare Ventil (11) einen durch die Recheneinheit (14) gesteuerten pulsatilen Gasstrom in die Kavität (8) ermöglicht.

Weitere Ausgestaltungen der Erfindung inklusive der dazugehörigen Betriebsverfahren werden nachfolgend beschrieben und sind teilweise Gegenstand von Neben- und Unteransprüchen.

**Grundzüge der Erfindung einfache Ausführungsformen**

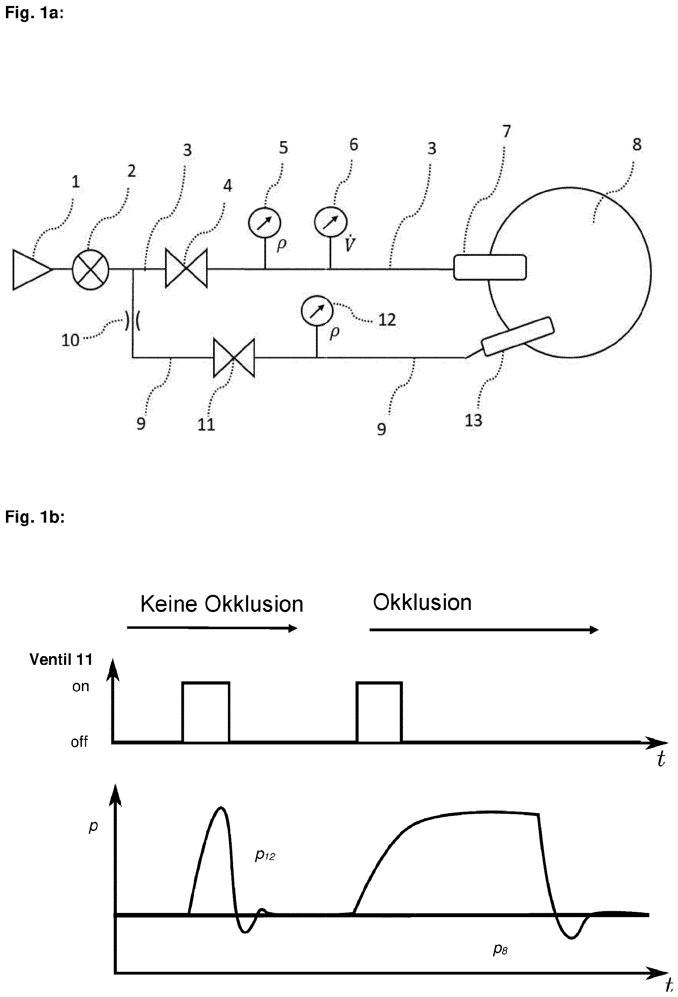

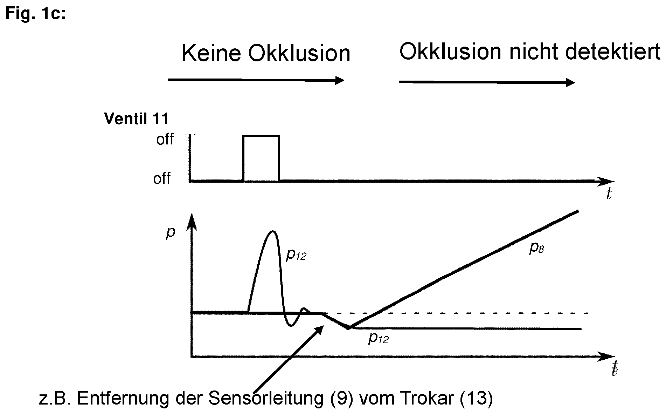

Die Erfindung wird grundsätzlich in Figur 1 dargestellt. Zu erkennen ist ein Gasanschluss für eine Gasflasche oder Hausgas, welcher zu einer ersten Druck- und Durchflussregeleinheit mit Proportionalventil führt. Die Leitung führt dann weiter zu einem steuerbaren Ventil. Die erste Druck- und Durchflussregeleinheit kann einen Gasfluss von bis zu 50 Liter/Min. bei einem Druck von 2 bis 3 bar liefern. Darüber hinaus zu erkennen ist ein Gasflusssensor, zur Messung des Gasstroms sowie ein erster Gasdrucksensor. Über den Schlauch und den Trokar führt die Zuführleitung in die Körperhöhle.

Zu erkennen ist weiterhin, dass zwischen der ersten Druck- und Durchflussregeleinheit und dem ersten steuerbaren Ventil eine Sensorleitung abzweigt. Diese Sensorleitung hat einen deutlich geringeren Querschnitt (3 bis 5 mm) und ist ausgelegt für einen Gasfluss weniger als 1 Liter/Min. bei dem oben bereits erwähnten Druck von 2 bis 3 bar. Bevorzugt ist der Gasfluss durch die Sensorleitung weniger als 0,5 Liter/Min., besonders bevorzugt weniger als 0,1 Liter/Min. Die Sensorleitung kann zum Zweck der Begrenzung des Gasflusses eine Drossel enthalten, optional kann die Drossel einstell- bzw. regelbar sein. Die Sensorleitung führt zunächst zu einem zweiten steuerbaren Ventil und anschließend zu einem zweiten Gasdrucksensor. Über einen zweiten Schlauch und einen zweiten Trokar führt die Sensorleitung ebenfalls in die Körperhöhle. Während des Betriebes kann über das zweite steuerbare Ventil ein diskontinuierlicher, pulsatiler Gasfluss über die Sensorleitung eingestellt werden, so wie es in Figur 1 b dargestellt ist. So kann beispielsweise ein einzelner Druckpuls von 20-50 mmHg für 1 bis 3 Sekunden in die Sensorleitung gegeben werden, gefolgt von einer Pulspause von 3 bis 20 Sekunden. Aufgrund des vergleichsweise geringen Gasflusses durch die Sensorleitung beeinflusst der Gasstrom durch die Sensorleitung den Druck in der Kavität praktisch nicht. Während eines Gaspulses steigt der vom zweiten Gasdrucksensor in der Sensorleitung gemessene Druck hingegen deutlich an und fällt nach dem Schließen des zweiten steuerbaren Ventils wieder ab, bis er mit dem Druck in der Körperhöhle identisch ist. Der tatsächliche Druck in der Körperhöhle entspricht daher dem Gleichgewichtsdruck während der Pulspausen. Im Falle einer Okklusion der Sensorleitung steigt der von dem zweiten Gasdrucksensor gemessene Druck an und bleibt für längere Zeit auf dem erhöhten Druckniveau (siehe Figur 1 b). Ein solches Druckverhalten zeigt klar die Okklusion der Sensorleitung an. In diesem Fall wir ein Alarmsignal ausgelöst, wenn der am zweiten Gasdrucksensor gemessene erhöhte Druck länger als das Doppelte der Pulsweite andauert.

Für die Detektion einer Okklusion der Sensorleitung ist daher zunächst der pulsatile Gasfluss durch die Sensorleitung entscheidend. Ohne die Gasflusspulse ist in dieser Ausführungsform der Erfindung eine Detektion eines Sensorleitungsverschlusses nicht möglich. Ferner ist der Vergleich der Messwerte von erstem Gasdrucksensor (5) und zweitem Gasdrucksensor (12) essentiell für die erfindungsgemäße Funktion der beschriebenen Vorrichtung und der damit durchgeführten Insufflationsverfahren.

Das ausgelöste Alarmsignal kann optischer und/oder akustischer Natur sein. Möglich ist auch eine automatische Begrenzung des Gaszuflusses bei der Insufflation zur Vermeidung eines zu hohen Druckes in der Kavität.

Der Aufbau des erfindungsgemäßen Insufflators macht aber auch eine geänderte Betriebsweise möglich, die nachfolgend beschrieben wird: In dieser Betriebsart wird ein kontinuierlicher Gasfluss durch die Sensorleitung eingestellt, durch konstante Öffnung des zweiten steuerbaren Ventils. Bei konstantem Gasfluss durch die Sensorleitung kann ein Druck am zweiten Gasdrucksensor ausgelesen werden, der vom Druck in der Kavität abhängt. Die Druckmessung am zweiten Gasdrucksensor muss allerdings, um den Druckverlust der Leitung und des Trokars zwischen dem zweiten Gasdrucksensor und der Kavität korrigiert werden. Hierzu gilt folgende Gleichung 1:

Gl. 1 $$p_{Kavität} = p_{Sensor} - p_{Sensorleitung}$$

Der Druckverlust in der Sensorleitung ist bei dem genannten geringen Gasfluss durch die Leitung näherungsweise konstant und kann durch Messung oder Berechnung ermittelt werden.

Bei dieser Betriebsweise steigt der vom zweiten Gasdrucksensor gemessene Druck an, wenn eine Okklusion eintritt. Der Druckanstieg um mindestens 20mmHg erfolgt innerhalb von ein bis zwei Sekunden und fällt annähernd genauso schnell wieder ab, wenn die Okklusion aufgelöst ist, z.B im Fall eines zwischenzeitlich abgeknickten Schlauches. Ein derartig schneller Druckanstieg bzw. Druckverlust kann nur im Fall einer Okklusion der Sensorleitung auftreten. Der Druckanstieg in der Körperhöhle im Falle einer Fehlsteuerung der Gaszufuhrleitung wäre deutlich langsamer.

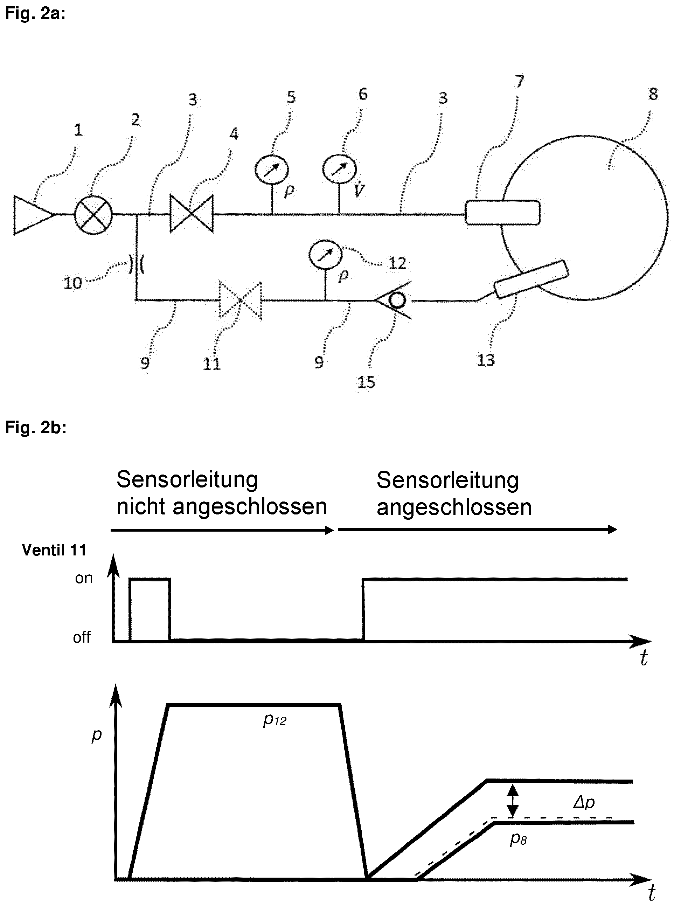

In einer weiteren Ausführungsform der Erfindung enthält die Sensorleitung zwischen dem zweiten Trokar und dem zweiten Gasdrucksensor noch ein Rückschlagventil, welches bei geringem Gegendruck seitens der Kavität von 8-12 mmHg öffnet. In speziellen Ausführungsformen der Erfindung kann das Rückschlagventil bereits bei geringeren Gegendrücken öffnen, beispielsweise bei 3mmHg bis 5 mmHg, oder sogar bei 1mmHg bis 3 mmHg. Auf diese Weise kann überprüft werden, ob die Sensorleitung ordnungsgemäß mit der Kavität verbunden ist und dass keine Okklusion vorliegt. Bei einer fehlenden Verbindung zur Kavität bzw. bei Auftreten einer Okklusion zwischen der Kavität und dem zweiten Sensor bleibt das Rückschlagventil geschlossen. Diese Situation kann dadurch erkannt werden, dass durch Öffnen des zweiten steuerbaren Ventils eine rasche Erhöhung des gemessenen Drucks am zweiten Gasdrucksensor auftritt, nicht jedoch am ersten Gasdrucksensor (siehe Figur 2b). Bei einer ordnungsgemäßen Verbindung steigt der gemessene Druck am zweiten Gasdrucksensor deutlich langsamer an. Gleichzeitig steigt auch der gemessene Druck am ersten Gasdrucksensor an. Auch in dieser Ausführungsform ist die Erzeugung eines pulsatilen Gasfluss durch die Sensorleitung notwendig. Ohne die Gasflusspulse ist eine Detektion eines Sensorleitungsverschlusses nicht möglich.

Ferner ist auch in diesem Fall die Kenntnis des Druckverlustes durch die Sensorleitung notwendig, um die Messung am zweiten Drucksensor entsprechend zu korrigieren (siehe Figur 2b).

In einer vereinfachten Ausführungsform der Erfindung (in den Figuren nicht dargestellt) führt die Sensorleitung mit dem daran angeschlossenen zweiten Gasdrucksensor über einen zweiten Trokar in die Kavität, ohne dass die Sensorleitung an die erste Druck- und Durchflussregeleinheit angeschlossen wäre. Das zweite steuerbare Ventil ist in diesem Fall entbehrlich. In dieser Ausführungsform kann eine Okklusion der Sensorleitung nur durch einen Vergleich der Messwerte zwischen dem ersten Gasdrucksensor und dem zweiten Gasdrucksensor erfolgen. Hierzu muss das erste steuerbare Ventil in der Zuführleitung geschlossen werden, damit der Messwert des ersten Gasdrucksensors nicht durch den Gaszustrom beeinflusst wird. Bei geschlossenem erstem Ventil, das heißt bei einer Insufflationspause, müssen beide Gasdrucksensoren den identischen Druck anzeigen, gegebenenfalls unter Berücksichtigung der Gasdruckverluste durch die Leitungen (siehe oben). Bei einer Abweichung der Druckwerte größer als 5 mmHg (in besonderen Fällen größer als 1mmHg) ist von einer Okklusion der Leitung oder einer anderen Störung des Gasflusses auszugehen.

**Beschreibung der Figuren**

Figur 1 zeigt die eingangs beschriebene, erfindungsgemäße Vorrichtung. Figur 1a zeigt den grundsätzlichen Aufbau mit den Komponenten des Anspruchs 1. Einzig die Recheneinheit ist nicht dargestellt. Durch das zweite steuerbare Ventil (11) wird ein pulsatiler Gasstrom in die Kavität (8) ermöglicht.

Figur 1b zeigt das Ergebnis der Druckmessung des zweiten Drucksensors ($p_{12}$) im Vergleich mit dem tatsächlichen Druck in der Kavität ($p_8$) in Abhängigkeit vom Gasfluss in der Sensorleitung. Die obere Linie zeigt die Stellung des zweiten steuerbaren Ventils (11), "on" oder "off". Die unteren Linien zeigen den gemessenen Druck am zweiten Drucksensor (12) bzw. in der Körperhöhle. Durch den Gaspuls in der Sensorleitung ergibt sich ein korrespondierender Druckpuls am zweiten Drucksensor (12) mit ungefähr gleicher Pulsweite. Im Fall eines Verschlusses der Sensorleitung (rechts im Bild) ergibt sich eine Druckerhöhung am zweiten Drucksensor (12), die deutlich länger andauert, als die Pulsweite des Druckpulses. Der tatsächliche Druck in der Körperhöhle ($p_8$) ist in diesem Beispiel konstant. Wenn die gemessene Druckerhöhung deutlich länger andauert, als die Pulsweite des Druckpulses aus dem zweiten steuerbaren Ventils (11), ohne dass eine entsprechende Druckerhöhung durch den ersten Drucksensor (5) registriert wird, kann auf einen Verschluss der Sensorleitung (9) geschlossen und ein Alarm ausgelöst werden.

Figur 1c zeigt den Druckverlauf im Fall einer Okklusion, nämlich dem Entfernen der Sensorleitung (9) vom zweiten Trokar ohne Pulsieren des zweiten steuerbaren Ventils. Der am zweiten Drucksensor gemessene Druck bleibt in diesem Fall konstant, auch wenn der Druck $p_8$ in der Kavität (8) steigt.

Figur 2a zeigt die Ausführungsform der Erfindung mit Rückschlagventil (15).

Figur 2b zeigt das Ergebnis der Messungen: Bei nicht angeschlossener Sensorleitung (9) an den zweiten Trokar (13) führt ein kurzer Druckpuls durch das zweite steuerbare Ventil (11) zu einer länger anhaltenden Druckerhöhung ($p_{12}$) am zweiten Drucksensor (12). Sobald die Sensorleitung (9) ordnungsgemäß an den zweiten Trokar (13) angeschlossen ist, verläuft der am zweiten Drucksensor (12) gemessene Druck ($p_{12}$) parallel zu dem tatsächlichen Druck in der Kavität (8). Die im Diagramm dargestellte Druckdifferenz $\Delta p$ ergibt sich aus dem Volumenstrom der Sensorleitung (9). Bei geschlossenem Ventil (11) wäre die Druckdifferenz geringer, aber aufgrund des Druckverlustes in der Leitung (siehe Gleichung 1) nicht gleich null. Diese Ausführungsform der Erfindung ist auch ohne das zweite steuerbare Ventil (11) möglich, welches deswegen optional ist. Bei Nichtvorhandensein des zweiten steuerbaren Ventils (11) ist allerdings nur die nicht ordnungsgemäße Verbindung von Sensorleitung (9) mit zweitem Trokar (13) detektierbar, nicht hingegen eine Okklusion zwischen Kavität (8) und zweitem Gasdrucksensor (12).

Die in den Figuren verwendeten Referenzzeichen haben folgende Bedeutung:

(1)   Gasanschluss für eine Gasflasche oder Hausgas

(2)   erste Druck- und Durchflussregeleinheit ausgestattet mit Proportionalventil

(3)   Zuführleitung

(4)   erstes steuerbares Ventil,

(5) erster Gasdrucksensor
(6) erster Gasflusssensor
(7) erster Trokar
(8) Kavität
(9) Sensorleitung
(10) Drossel (optional)
(11) zweites steuerbares Ventil
(12) zweiter Gasdrucksensor
(13) zweiter Trokar
(14) Recheneinheit
(15) Rückschlagventil

**Patentansprüche**

1. Insufflator für die minimal-invasive Chirurgie, enthaltend

   a) Gasanschluss für eine Gasflasche oder Hausgas (1)
   b) erste Druck- und Durchflussregeleinheit (2) ausgestattet mit Proportionalventil,
   c) Zuführleitung (3) mit einem Querschnitt von 5,5 bis 15 mm, ausgelegt für einen Gasfluss von 0-50 L/min bei 2-3 bar, mit erstem steuerbarem Ventil (4), erstem Gasdrucksensor (5), erstem Gasflusssensor (6) und Anschluss an einen ersten Trokar (7) in eine Kavität (8), **dadurch gekennzeichnet, dass** der Insufflator ferner enthält
   d) Sensorleitung (9) mit einem Querschnitt von 2 bis 5 mm mit einer optionalen Drossel (10), ausgelegt für einen Gasfluss von weniger als 1 L/min bei 2-3 bar, mit einem zweiten steuerbaren Ventil (11) und einem zweiten Gasdrucksensor (12) und Anschluss an einen zweiten Trokar (13) in die Kavität (8),
   e) Recheneinheit (14) zur Steuerung der Insufflation und Auswertung der Messdaten von erstem Gasdrucksensor (5) und zweitem Gasdrucksensor (12),
   wobei die Sensorleitung (9) an die erste Druck- und
   Durchflussregeleinheit (2) angeschlossen ist und wobei das zweite steuerbare Ventil (11) einen durch die Recheneinheit (14) gesteuerten pulsatilen Gasstrom in die Kavität (8) ermöglicht.

2. Insufflator für die minimal-invasive Chirurgie gemäß Anspruch 1, ferner enthaltend ein Rückschlagventil (15), welches bereits bei einem geringen Gegendruck von 8 bis 12 mmHg seitens der Kavität öffnet, welches in der Sensorleitung zwischen dem zweiten Trokar (13) und dem zweiten Gasdrucksensor (12) positioniert ist.

3. Insufflator für die minimal-invasive Chirurgie, gemäß Anspruch 1, wobei die Recheneinheit (14) ausgelegt ist für eine Korrektur der Druckverluste der Zuführleitung (3) mit erstem Trokar (7) und der Sensorleitung (9) mit zweiten Trokar (13) und einen Vergleich der korrigierten Messwerte von erstem Drucksensor (5) und zweitem Drucksensor (6).

**Claims**

1. Insufflator for minimally invasive surgery, comprising

   a) gas connection for a gas cylinder or domestic gas (1)
   b) first pressure and flow control unit (2) equipped with a proportional valve,
   c) supply line (3) with a cross-section of 5.5 to 15 mm, designed for a gas flow of 0-50 L/min at 2-3 bar, with a first controllable valve (4), first gas pressure sensor (5), first gas flow sensor (6), and connection to a first trocar (7) in a cavity (8), **characterized in that** the insufflator further comprises
   d) a sensor line (9) with a cross-section of 2 to 5 mm with an optional throttle (10), designed for a gas flow of less than 1 L/min at 2-3 bar, with a second controllable valve (11) and a second gas pressure sensor (12), and connection to a second trocar (13) in the cavity (8),
   e) a computing unit (14) for controlling the insufflation and evaluating the measurement data from the first gas pressure sensor (5) and the second gas pressure sensor (12),

   wherein the sensor line (9) is connected to the first pressure and flow control unit (2) and wherein the second controllable valve (11) enables a pulsatile gas flow into the cavity (8) controlled by the computation unit (14).

2. Insufflator for minimally invasive surgery according to claim 1, further comprising a check valve (15) which opens at a low back pressure of 8 to 12 mmHg from the cavity, and which is positioned in the sensor line between the second trocar (13) and the second gas pressure sensor (12).

3. Insufflator for minimally invasive surgery according to claim 1, wherein the computing unit (14) is designed to correct the pressure losses of the supply line (3) with the first trocar (7) and the sensor line (9) with the second trocar (13) and to compare the corrected measured values of the first pressure sensor (5) and the second pressure sensor (6).

**Revendications**

1. Insufflateur pour la chirurgie mini-invasive, comprenant

a) un raccord de gaz pour une bouteille de gaz ou un réseau de gaz central (1)

b) une première unité de régulation de pression et de débit (2) équipée d'une vanne proportionnelle,

c) une conduite d'alimentation (3) présentant une section de 5,5 à 15 mm, conçue pour un débit de gaz de 0 à 50 L/min sous une pression de 2 à 3 bar, avec une première vanne commandable (4), un premier capteur de pression de gaz (5), un premier capteur de débit de gaz (6) et un raccordement à un premier trocart (7) dans une cavité (8), **caractérisé en ce que** l'insufflateur comprend en outre

d) une conduite de capteur (9) présentant une section de 2 à 5 mm comportant éventuellement un organe d'étranglement (10), conçue pour un débit de gaz inférieur à 1 L/min sous une pression de 2 à 3 bar, avec une deuxième vanne commandable (11), un deuxième capteur de pression de gaz (12) et un raccordement à un deuxième trocart (13) dans la cavité (8),

e) une unité de calcul (14) destinée à commander l'insufflation et à évaluer les données de mesure du premier capteur de pression de gaz (5) et du deuxième capteur de pression de gaz (12),

la conduite de capteur (9) étant reliée à la première unité de régulation de pression et de débit (2) et la deuxième vanne commandable (11) permettant un flux de gaz pulsé dans la cavité (8), commandé par l'unité de calcul (14).

2. Insufflateur pour la chirurgie mini-invasive selon la revendication 1, comprenant en outre un clapet anti-retour (15) qui s'ouvre dès qu'une faible contre-pression de 8 à 12 mmHg est exercée par la cavité, lequel est positionné dans la conduite de capteur entre le deuxième trocart (13) et le deuxième capteur de pression de gaz (12).

3. Insufflateur pour la chirurgie mini-invasive selon la revendication 1, dans lequel l'unité de calcul (14) est conçue pour corriger les pertes de pression de la conduite d'alimentation (3) avec le premier trocart (7) et de la conduite de capteur (9) avec le deuxième trocart (13), et pour comparer les valeurs de mesure corrigées du premier capteur de pression (5) et du deuxième capteur de pression (6).

**Fig. 1a:**

**Fig. 1b:**

**Fig. 1c:**

Keine Okklusion    Okklusion nicht detektiert

**Ventil 11**

off

off

$t$

$p$

$p_{12}$

$p_8$

$p_{12}$

$t$

z.B. Entfernung der Sensorleitung (9) vom Trokar (13)

**Fig. 2a:**

**Fig. 2b:**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202004021703 U1
- US 5908402 A